# EUROPEAN PATENT APPLICATION

(11) **EP 4 450 508 A1**
(43) Date of publication of application: **23.10.2024**
(21) Application number: 21967736.6
(22) Date of filing: 17.12.2021
(51) Int. Cl.: C07D 513/04, C07D 211/74

(54) **INTERMEDIATE OF EDOXABAN TOSYLATE AND PREPARATION METHOD THEREFOR**

(30) Priority: 14.12.2021 CN 202111529593
(71) Applicant: Zhejiang Jiuzhou Pharmaceutical Co., Ltd., Jiaojiang District Taizhou City Zhejiang 318000 (CN)
(72) Inventor: MEI, Yijiang, Taizhou, Zhejiang 318000 (CN); LIU, Shengmin, Taizhou, Zhejiang 318000 (CN); LI, Yonggang, Taizhou, Zhejiang 318000 (CN); FAN, Jinmin, Taizhou, Zhejiang 318000 (CN); HU, Kai, Taizhou, Zhejiang 318000 (CN); GAO, Shizhao, Taizhou, Zhejiang 318000 (CN)
(74) Representative: HGF
(86) International application number: PCT/CN2021/139089
(87) International publication number: WO 2023/108602

(57) **Abstract**

Disclosed are an intermediate of edoxaban tosylate and a preparation method therefor, having the advantages that the preparation process is simple, reaction conditions are mild, the cost is low, and raw materials are easily to be obtained. The method comprises the synthesis steps of: substitution reaction, thiolation reaction, cyclization reaction, esterification reaction with alcohol to obtain a compound represented by formula (V), and then reacting to obtain a compound represented by formula (VI), or directly reacting the compound represented by formula (IV) under the effect of alcohol and acid, to obtain the compound represented by formula (VI), and subjecting the compound represented by formula (VI) to alkali hydrolysis and acid neutralization to obtain a compound represented by formula (VII). The reaction formula is as follows:

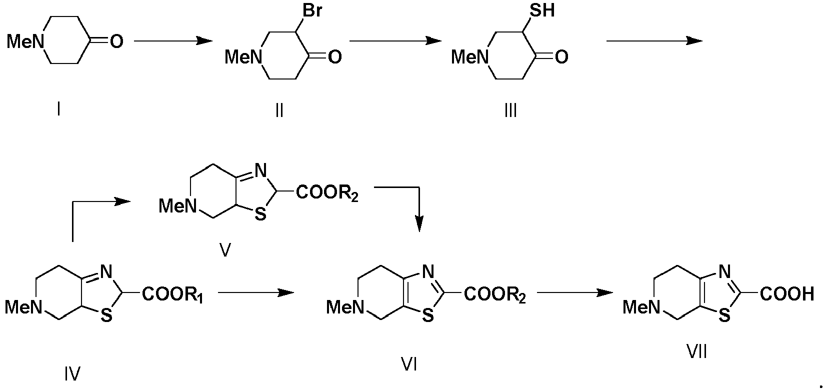

## Description

### FIELD OF THE INVENTION

This invention relates to the field of organic chemical synthesis and, in particular, relates to intermediate of edoxaban tosylate and a preparation method therefor.

### BACKGROUND OF THE INVENTION

Edoxaban tosylate is a direct anticoagulant factor Xa inhibitor used for the treatment of total knee arthroplasty, total hip replacement, or venous thromboembolism in patients following hip fracture surgery. The present invention relates to methods for the preparation of their pharmacologically acceptable salts or their hydrates.

Edoxaban tosylate was developed by Daiichi Sankyo, Inc. and was approved for marketing by the Japan Pharmaceutical and Medical Devices Agency (PMDA) on April 22, 2011, followed by the U.S. Food and Drug Administration (FDA) on January 8, 2015, and by the European Medicines Agency (EMA) on June 19, 2015, and was approved by the National Drug Administration (NMPA) on December 25, 2018, for marketing. It is marketed in Japan by Daiichi Sankyo Co. Ltd. under the trade name Lixiana^{®} as an oral tablet.

At present, there are many synthetic process routes for edoxaban tosylate in the market, but most of them are synthesized by synthesizing its key intermediate with the chemical name 4,5,6,7-tetrahydro-5-methyl-thiazolo[5,4-c] pyridine-2-carboxylic acid. It therefore makes a crucial synthetic study for both edoxaban tetra benzenesulfonate and its key intermediate, which the key intermediate has the following structural formula:

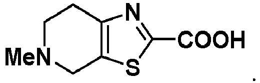

The main synthetic routes for the preparation of 4,5,6,7-tetrahydro-5-methyl-thiazolo[5,4-C] pyridine-2-carboxylic acid were reported and disclosed in literature which contain two schemes and are listed as follows:

### 1) 109C6x Synthesis route I (US2005119486A1)

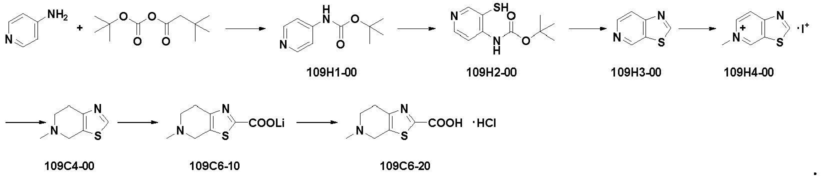

The route takes 4-aminopyridine as the raw material and comprised the following steps, using the raw material reacting with Boc-anhydride to obtain 109H1-00, under the action of n-butyl lithium and S monomer to obtain compound 109H2-00, carried out ring-closing reaction with formic acid to obtain compound 109H3-00, then reacting with iodomethane to obtain the corresponding pyridinium quaternary ammonium compounds 109H4-00, and carrying out reduction reaction with borohydride to obtain compound 109C4-00, and then preparing the corresponding carboxylated lithium salts with n-butyl lithium and carbon dioxide gas to obtain the corresponding carboxylated lithium salts 109C6-10.

Finally, the mixture was neutralized by hydrochloric acid to form a salt of compound 109C6-10 with formula name 109C6-20. In the synthetic route, expensive and easily flammable n-butyllithium was used twice, easily flammable and explosive S monomer and low boiling point, expensive and toxic iodomethane reagent were also used. From the point of view of industrial safety, there exist hidden risks, and the synthetic route is not conducive to controlling the cost. It is not suitable for industrialized production.

### 2) 109C6x Synthesis route (US9233980B2)

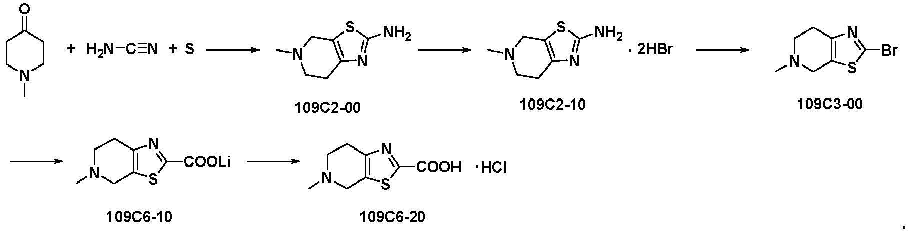

In this synthetic route, N-methyl-4-piperidone was used as starting material and cyclized with aqueous monocyanamide and S monomer to obtain the corresponding thiazolamine compound 109C2-00, and the bromine substituent 109C3-00 was obtained by diazotization of sodium nitrite and hydrobromic acid, and the lithium carboxylate was obtained by reacting 109C3-00 with n-butyl chloride and carbon dioxide gas under low-temperature conditions, and the compound 109C6-20 which was a salt form was finally obtained by neutralizing with hydrochloric acid. In the synthetic, easily flammable and explosive S monomer and expensive and easily flammable n-butyllithium were used. There exist hidden production safety hazards, and it is not conducive to controlling the cost. It is not suitable for industrialized-scale production.

In view of this, it is an object of the present invention to provide a method of preparing 4,5,6,7-tetrahydro-5-methyl-thiazolo[5,4-c] pyridine-2-carboxylic acid. The method has the advantages of easily to prepare, mild reaction conditions, and low cost. And the raw material should be easily available. In that way, it can overcome the above deficiencies existing in the prior arts.

### SUMMARY OF THE INVENTION

The present disclosure provides an intermediate for the preparation of edoxaban tosylate and a method for its preparation, and the method has the advantages of a simple process, mild reaction conditions, low cost, and easy availability of the raw materials. In order to achieve the objectives of the present disclosure, the present disclosure provides the following technical solutions:

In the first aspect, the present disclosure provides an intermediate formula (II) for the preparation of edoxaban tosylate with the following structural formula:

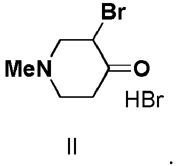

In a second aspect, the present disclosure provides a method of preparing an intermediate formula (II) of edoxaban tosylate, the definitions of the synthesis step of the compound of formula (II) is described as comprising: a substitution reaction with bromine using the compound N-methyl-4-piperidone as a raw material to produce the compound 3-bromo-N-methyl-4-piperidone shown in formula (II).

As a preference, the reaction temperature during the substituting reaction with the bromine is 20-25°C, at the said temperature, the occurrence of side reactions can be effectively prevented, so that the yield of the compound shown in the final formula (II) is maintained at a high value.

In a third aspect, the present disclosure provides an intermediate formula (III) for the preparation of edoxaban tosylate having the structural formula as follows:

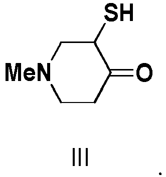

In a fourth aspect, the present disclosure provides a method of preparing an intermediate formula (III) of edoxaban tosylate, the synthesis step of the compound of formula (III) is described as comprising: the compound shown in formula (II) is subjected to a thiolation reaction with sodium sulfide to obtain the compound shown in formula (III) with the following reaction formula:

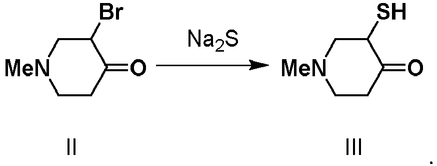

As a preference, a reaction temperature of -5 to 0°C and a reaction time of 20 to 60 min are maintained during the thiolation reaction.

### BRIEF DESCRIPTION OF DRAWINGS

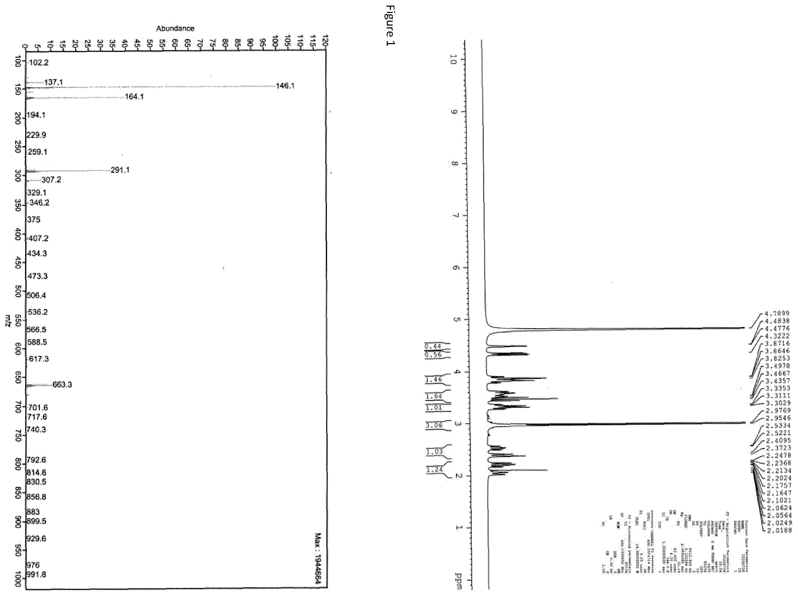

Figure 1 shows the H-NMR spectrum of compound (II).
Figure 2 shows the mass spectrometry spectrum of compound (III).

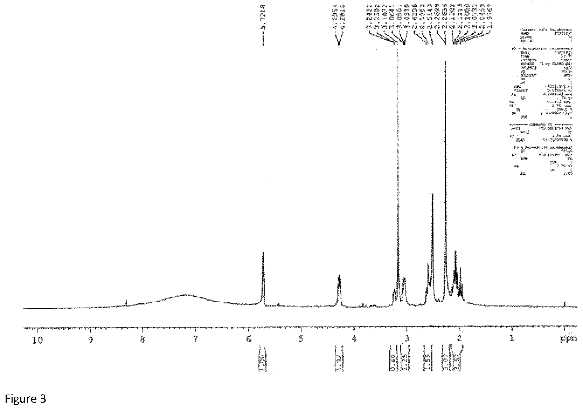

Figure 3 shows the H-NMR spectrum of compound (IV-1).

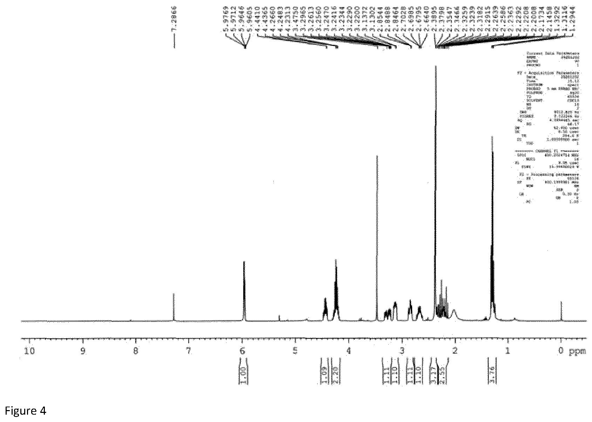

Figure 4 shows the H-NMR spectrum of compound (V-1).

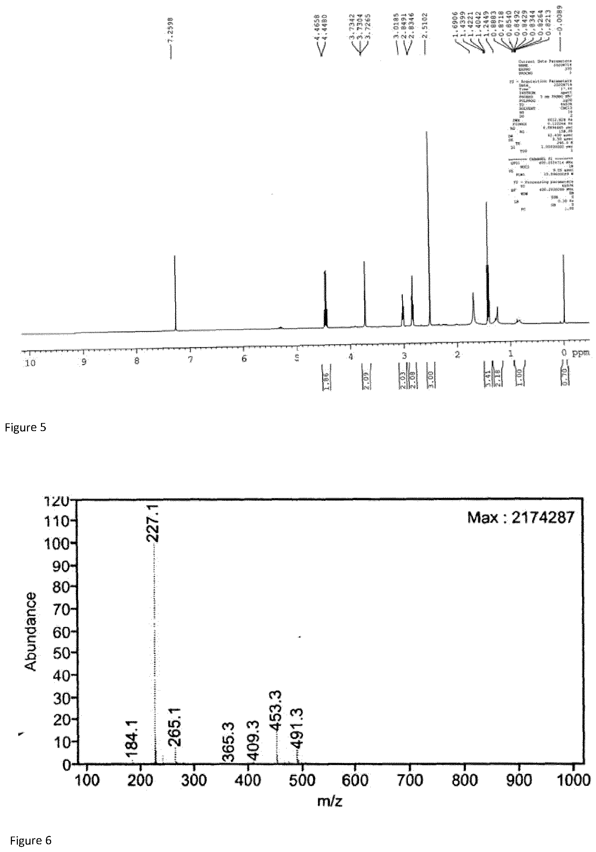

Figure 5 shows the H-NMR spectrum of compound (VI-1).
Figure 6 shows the mass spectrometry spectrum of compound (VI-1).

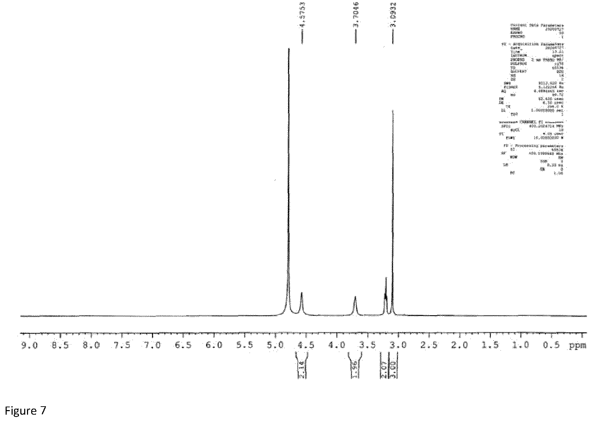

Figure 7 shows the H-NMR spectrum of compound (VII).

### DETAILED DESCRIPTION OF THE INVENTION

In a fifth aspect, the present disclosure provides an intermediate formula (IV) for the preparation of edoxaban tosylate with the following structural formula: 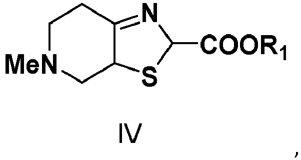 R₁=NH₄ or H.

In a sixth aspect, the present disclosure provides a method of preparing an intermediate formula (IV) of edoxaban tosylate, in which a compound N-methyl-4-piperidone is used as raw material, and a substitution reaction is carried out with bromine to produce the compound shown in formula (II),

The compound shown in formula (II) undergoes a thiolation reaction with sodium sulfide to obtain the compound shown in formula (III),

The compound shown in formula (III) was subjected to cyclization reaction with glyoxalic acid to obtain the compound shown in formula (IV) with the following reaction formula: 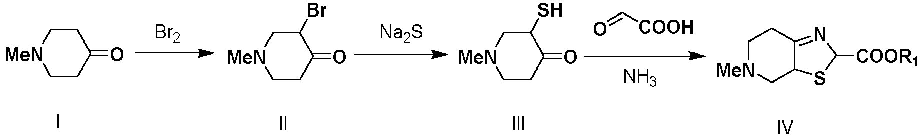 R₁=NH₄ or H.

In a seventh aspect, the present disclosure provides an intermediate formula (IV-I) for the preparation of edoxaban tosylate with the following structural formula:

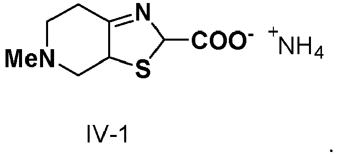

In an eighth aspect, the present disclosure provides a method of preparing an intermediate formula (IV-I) of edoxaban tosylate, wherein the synthesis step of the compound shown in formula (IV-I) is described as comprising: the compound shown in formula (III) is subjected to a cycloaddition reaction with glyoxalic acid as well as ammonia to obtain the compound N-methyl-4-piperidino-dihydrothiazolecarboxylic acid ammonium salt, shown in formula (IV-I), with the following reaction formula:

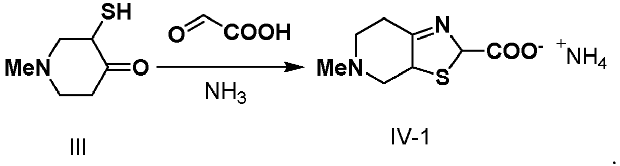

In a ninth aspect, an intermediate formula (IV-2) for the preparation of edoxaban tosylate, with the following structural formula:

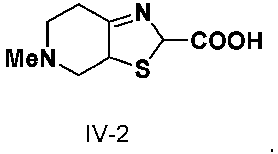

In a tenth aspect, a method of preparing an intermediate formula (IV-2) of edoxaban tosylate, wherein the synthesis step of the compound shown in formula (IV-2) is described as comprising: acidification of the compound shown in formula (IV-I) to obtain the compound shown in formula (IV-2).

As a preference, during the cyclization reaction, the reaction temperature is maintained at 20^{~}25°C and the reaction is stirred for 18^{~}36h.

In an eleventh aspect, the present disclosure provides an intermediate formula (V) for the preparation of edoxaban tosylate, with the following structural formula: 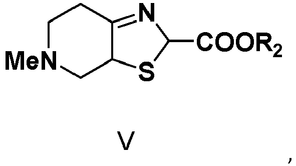 R₂ is selected from C1-C6 aliphatic hydrocarbons, phenyl, or substituted phenyl.

In a twelfth aspect, the present disclosure provides a method for preparing an intermediate formula (V) of edoxaban tosylate, the synthesis step of the compound shown in formula (V) is described as comprising: the compound shown in formula (IV) and an alcohol under acid-catalyzed esterification reaction catalyzed by an acid to obtain the compound shown in formula (V) with the following reaction formula: 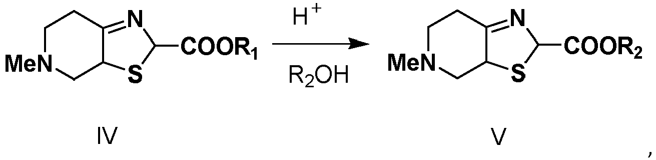 R₁ is selected from NH₄ or H, R₂ is selected from C1-C6 aliphatic hydrocarbons, phenyl, or substituted phenyl.

As a preference, the definition of alcohol is any one of methanol, ethanol, propanol, isopropanol, phenol, and the like.

As a preference, the definition of the acid is concentrated sulfuric acid.

As a preference, during the esterification reaction, the reaction temperature is maintained at 20-25°C, the reaction is stirred for 8-12h, and after the reaction, sodium bicarbonate is used to neutralize to a pH of 7-8.

In a thirteenth aspect, the present disclosure provides an intermediate formula (V-I) for the preparation of edoxaban tosylate, with the following structural formula:

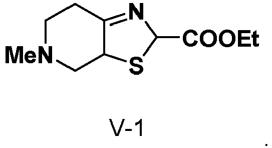

In a fourteenth aspect, the present disclosure provides a method for preparing an intermediate formula (V-I) of edoxaban tosylate, the synthesis step of the compound shown in formula (V-I) is described as comprising: the compound shown in formula (IV-I) and ethanol under acid-catalyzed esterification reaction catalyzed by acid to obtain the compound of formula (V-I) with the following reaction formula:

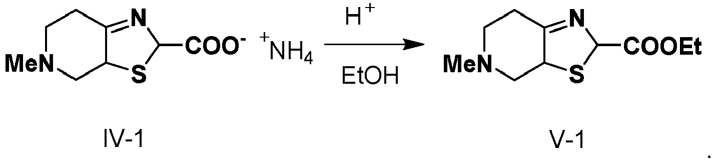

In a fifteenth aspect, the present disclosure provides an intermediate formula (VI) for the preparation of edoxaban tosylate with the following structural formula: 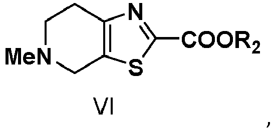 R₂ is selected from C1-C6 aliphatic hydrocarbons, phenyl, or substituted phenyl.

In a sixteenth aspect, the present disclosure provides a method of preparing an intermediate formula (VI) of edoxaban tosylate, the synthesis step of the compound shown in formula (VI) is described as comprising: The compound shown in formula (V) is obtained from the esterification reaction of the compound shown in formula (IV) with an alcohol, which is then reacted to obtain the compound shown in formula (VI), or the compound shown in formula (VI) is obtained directly from the reaction of the compound shown in formula (IV) in the presence of an alcohol and an acid, with the following reaction formula: 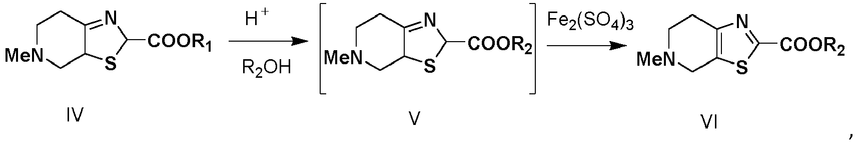 or, 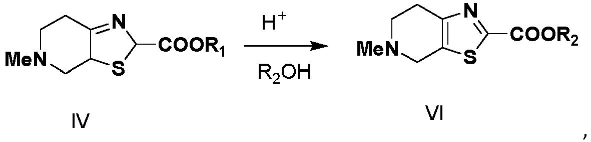 R₁ is selected from NH₄ or H, R₂ is selected from C1-C6 aliphatic hydrocarbons, phenyl, or substituted phenyl.

In a seventeenth aspect, the present disclosure provides an intermediate formula (VI-I) for the preparation of edoxaban tosylate with the following structural formula:

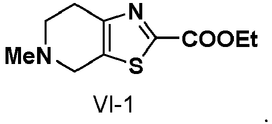

In an eighteenth aspect, the present disclosure provides a method of preparing an intermediate formula (VI-1) of edoxaban tosylate, the synthesis step of the compound shown in formula (VI-1) is described as comprising: The compound shown in formula (V-I) is obtained by esterification of the compound shown in formula (IV-I) with ethanol, and then reacted to obtain the compound shown in formula (VI-I), or the compound shown in formula (VI-I) is obtained directly by the reaction of the compound shown in formula (IV-I) in the presence of ethanol and acid, with the following structural formula: 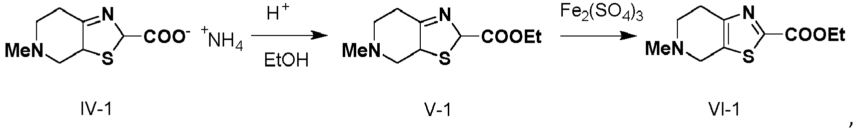 or,

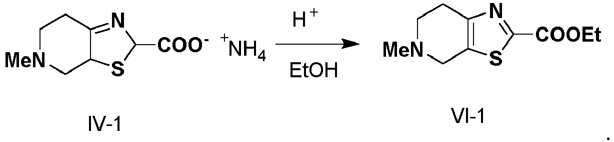

In a nineteenth aspect, the present disclosure provides a method of preparing an intermediate formula (VII) of edoxaban tosylate, the synthesis step of the compound shown in formula (VII) is described as comprising:
The compound N-methyl-4-piperidone was used as a raw material for the substitution reaction with bromine to produce the compound shown in formula (II),
The compound shown in formula (II) undergoes a thiolation reaction with sodium sulfide to obtain the compound shown in formula (III),
The compound shown in formula (III) was subjected to a cyclization reaction with glyoxalic acid to obtain the compound shown in formula (IV),
The compound shown in formula (IV) undergoes esterification reaction with alcohol to obtain the compound shown in formula (V), and then reacts to obtain the compound shown in formula (VI), or the compound shown in formula (VI) is obtained directly by the reaction of the compound shown in formula (IV) under the action of alcohol and acid, the compound shown in formula (VI) is obtained by hydrolysis by alkali and neutralization by acid to obtain the compound shown in formula (VII).

The reaction formula is as follows: 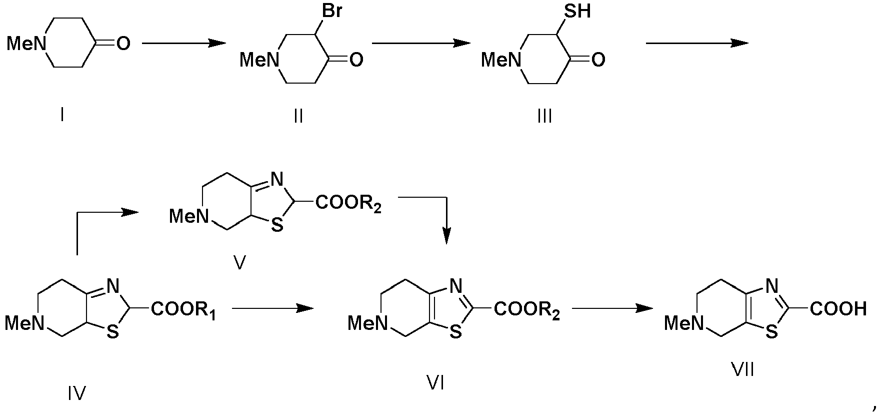 R1 is selected from NH₄ or H, R₂ is selected from C1-C6 aliphatic hydrocarbons, phenyl, or substituted phenyl.

In a twentieth aspect, the present disclosure provides a method of preparing an intermediate formula (VII) of edoxaban tosylate, the synthesis step of the compound shown in formula (VII) is described as comprising:
The compound N-methyl-4-piperidone was used as a raw material for the substitution reaction with bromine to produce the compound shown in formula (II),
The compound shown in formula (II) undergoes a thiolation reaction with sodium sulfide to obtain the compound shown in formula (III),
The compound shown in formula (III) was subjected to a cyclization reaction with glyoxalic acid to obtain the compound shown in formula (IV-I),
The compound shown in formula (IV) undergoes esterification with an alcohol to obtain the compound shown in formula (V-I), which is then reacted to obtain the compound shown in formula (VI-I), or the compound shown in formula (VI-I) is obtained directly from the reaction of the compound shown in formula (IV-I) in the presence of an alcohol and an acid,
The compound shown in formula (VI-I) is hydrolyzed by alkali and neutralized by acid to obtain the compound shown in formula (VII).

The reaction formula is as follows:

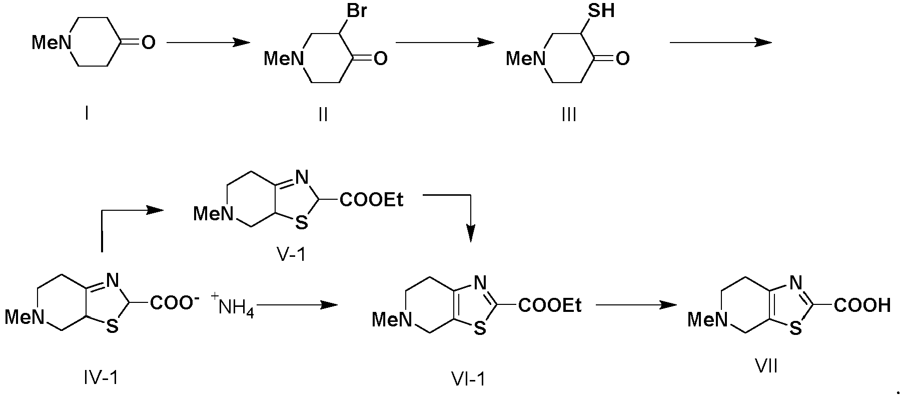

Therefore, the present disclosure has the following beneficial effects:
(1) Simple and easy to implement: the synthetic route used in the present disclosure is easy to prepare the raw materials, and the stability of the product of each step is greatly increased, the stability of the transportation, storage, and usage of the factory production has been greatly increased, which makes the reaction of the intermediates in the quality control more convenient and reduces the requirements of the equipment. In addition, the synthetic route is not involved in complex reactions. It can be concluded that the entire synthetic route has the advantages of not only being simple but also easy to operate.
(2) Low cost: The synthetic route of the present disclosure does not use valuable catalysts or auxiliary reagents in the reaction steps in which various raw materials are involved, to ensure that the wastes generated by the various steps of the process are less emission and easy to dispose of, which makes the reaction both green and environmental protection at the same time and greatly reduces the cost,
(3) High yield: In the new intermediate and its preparation method using edoxaban tosylate of the present disclosure, the yield of each step of the reaction is high, and the post-processing is simple and convenient, which is more conducive to the regulation during large-scale production.

### EXAMPLES

### Example 1: The edoxaban tosylate intermediate 4,5,6,7-tetrahydro-5-methyl-thiazolo[5,4-c] pyridine-2-carboxylic acid of the present invention was obtained using the following process steps.

### (1) Synthesis of compound (II)

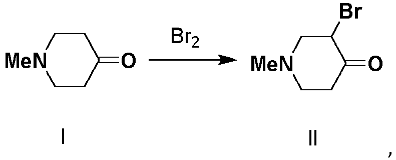

Into the reaction flask, 40g of glacial acetic acid was added, the temperature was kept below 25°C, 22.6g (199.7 mmol) of N-methyl-4-piperidone was added dropwise, 34g (201.7 mmol) of 48% aqueous hydrobromic acid solution was added dropwise and 40g of glacial acetic acid was prepared, the temperature was kept below 20°C, 32g (200.2 mmol) of bromine and 30g of glacial acetic acid were added dropwise, after finishing dropping, the temperature of the reaction mixture was kept at 20-25°C and stirring the reaction mixture overnight. After that, filtration, the filter cake was leached with ethyl acetate and then collected the solid, drying, to obtain 52 g of compound II, with a yield of 95.4%.

### (2) Synthesis of compound (III)

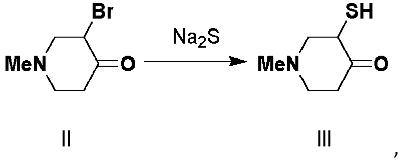

The reaction flask was added 10 g of water and 3.7 g (22.0 mmol) of Na₂S.5H₂O, stirred to dissolve, the temperature was lowered to -5-0 ° C,3-bromo-N-methyl-4-piperidinone 5 g (18.3 mmol) was added in portions, after finishing dropping, maintaining the temperature at -5-0 ° C, stirring the reaction for 0.5 h. The reaction mixture was filtrated and the filter cake was washed with water, the solids were collected, and dried, to obtain 2.4 g of compound III, with a yield of 90%.

### (3) Synthesis of compound (IV-1)

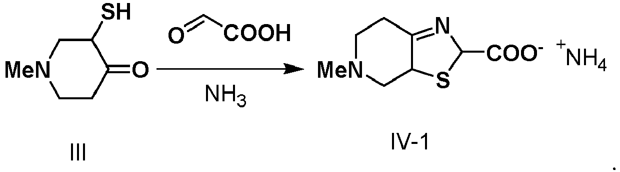

Into the reaction flask was added 45 g of methanol, 2.5 g (27 mmol) of glyoxalate monohydrate and 4 g (27 mmol) of Compound III, stirring to dissolve, the temperature was controlled below 30 °C, dropwise addition of 7 M ammonia/methanol solution 16 ml (108 mmol), after finishing dropping, the temperature of reaction mixture was maintained at 20-25 °C and stirring the reaction mixture for 24 hours, the reaction solution was concentrated at 40 °C under reduced pressure to obtain 6.4 g of solid crude compound IV-1 in 50% yield.

### (4) Synthesis of compound (V-1)

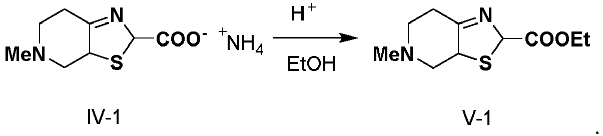

Into the reaction flask was added ethanol 10 ml, compound IV crude 0.6 g, stirring to dissolve, temperature controlled at 0-5 °C, dropwise addition of concentrated sulfuric acid 0.5 ml (5 mmol), after finishing dropping, slowly rising the temperature to 20-25 °C by insulation. By stirring reaction mixture for 10 hours, using saturated sodium bicarbonate solution to adjust and neutralize pH = 7-8, the reaction mixture was concentrated at 50 °C under reduced pressure, the concentrated mixture was stirred by adding methanol 10 ml, filtration, the filtrate was concentrated at 40 °C under reduced pressure, to obtain the compound V-1 crude 0.6g.

### (5) Synthesis of compound (VI-1)

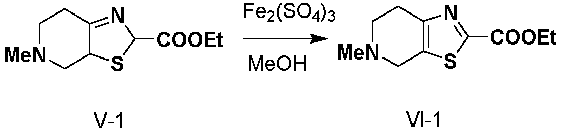

Into the reaction flask was added methanol 20ml, compound IV-1 crude 0.6g, Fe₂(SO₄)₃ solid 0.6g, stirring to dissolve, heating temperature and refluxing reaction for 10 hours, filtration. The filtrate was concentrated at 50 °C under reduced pressure, the concentrate mixture was loaded on a silica gel column, solvent methanol / dichloromethane = 1:20 ratio of elution and purification, to obtain 0.2 g of compound VI-1.

### (6) Synthesis of compound (VII)

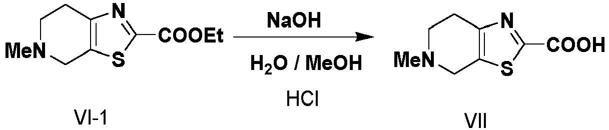

Into the reaction flask were added 1.3 g (5.8 mmol) compound VI-1, methanol 10 ml, stirring the mixture to dissolve, the temperature was reduced to 0-5 °C, dropwise addition of 1 M sodium hydroxide solution 6 ml (6 mmol), after finishing dropwise addition , maintaining the temperature at 0-5 °C by heat preservation and stirring the mixture for 1.5 hours, Adjusting the pH of the reaction solution with 0.5 M dilute hydrochloric acid to pH = 3-4, the temperature of the reaction solution was controlled at 40-45 °C under reduced pressure, after evaporating the methanol, the concentrate was filtered, the filter cake was washed with ethanol 3 ml/ water 6 ml mixed solution, , then the filter cake was dried to obtain compound VII.

### Example 2:

The edoxaban tosylate intermediate 4,5,6,7-tetrahydro-5-methyl-thiazolo[5,4-c] pyridine-2-carboxylic acid of the present invention was obtained using the following process steps.

### (1) Synthesis of compound (II)

Into the reaction flask, was added 30 g of glacial acetic acid, the temperature was controlled below 25°C, dropwise addition of N-methyl-4-piperidone 17.0 g (149.8 mmol), dropwise addition of 48% aqueous hydrobromic acid solution of 25.5 g (151.3 mmol) with 30 g of glacial acetic acid prepared solution, temperature was controlled below 20°C, dropwise addition of bromine 24 g (150.2 mmol) with prepared glacial acetic acid 22.5 g and the temperature was controlled at 20-25°C. After dropping the solution, it was stirred overnight while keeping temperature warm at 20-25°C and then filtered, the filter cake was washed with ethyl acetate, the solid was collected, and dried, to obtain 40 g of Compound II in 97.9% yield.

### (2) Synthesis of compound (III)

Into the reaction flask was added 10g of water and 3.36g (20.0 mmol) of Na₂S.5H₂O, stirring to dissolve, the temperature was lowered to -5-0 ° C, adding 3-bromo-N-methyl-4-piperidinone 4.55g (16.7 mmol) in batches, after finishing dropping, the temperature was kept at -5-0 ° C by insulating and stirring the reaction mixture for 1 hour, filtration, the filter cake was washed with water, the solid was collected, dried, to obtain 2.21g Compound III, with a yield of 91.2%.

### (3) Synthesis of compound (IV-1)

Into the reaction flask were added 45 g of methanol, 2.5 g (27 mmol) of glyoxalate monohydrate and 4 g (27 mmol) of Compound III, stirring to dissolve, the temperature was controlled below 30 °C, dropwise addition of 7 M ammonia/methanol solution 16 ml (108 mmol), after finishing dropwise addition, the reaction mixture was stirred at 20-25 °C for 36 hours while maintaining the temperature warm, and the reaction solution was concentrated at 40 °C under reduced pressure to give 6.4 g of solid crude compound IV-1 in 50% yield.

### Synthesis of compound (V-2)

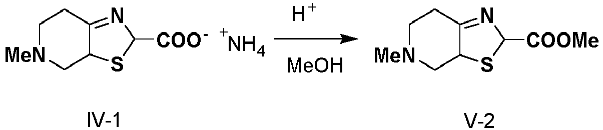

Into the reaction bottle were added methanol 10 ml, compound IV crude 0.5 g, stirring to dissolve, temperature control at 0-5 °C, dropwise addition of concentrated sulfuric acid 0.5 ml (5 mmol), after finishing dropping, slowly rising the temperature to 20-25 °C by insulating and stirring the reaction mixture for 8 hours, using saturated sodium bicarbonate solution to adjust and neutralize pH = 7-8, the temperature of the reaction was kept at 50 °C under reduced pressure concentration, adding methanol(10 ml) into the concentrate and stirring, filtration, the filtrate was concentrated at 40 °C under reduced pressure to obtain compound V-2 crude 0.5g.

### (5) Synthesis of compound (VI-2)

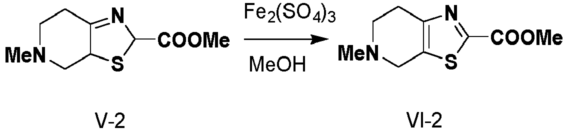

Into the reaction flask were added methanol 20ml, compound IV-2 crude 0.6g, Fe₂(SO₄)₃ solid 0.6g, stirring to dissolve, heating temperature to reflux reaction for 8 hours, filtration, the filtrate was concentrated at 50 °C under reduced pressure, the concentrated mixture was loaded on a silica gel column, solvent methanol / dichloromethane = 1:20 ratio of elution and purification, to obtain 0.2 g of compound VI-2.

### (6) Synthesis of compound (VII)

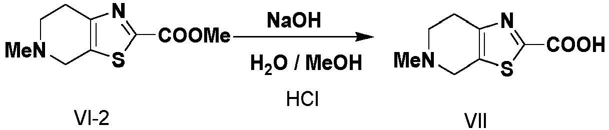

Into the reaction flask were added 1.17 g (5.5 mmol) compound VI-2, methanol 10 ml stirring to dissolve, the temperature was lowered to 0-5 ° C, dropwise addition of 1M sodium hydroxide solution 6 ml (6 mmol), after finishing dropwise addition, maintaining the temperature at 0-5 ° C by insulation and stirring the reaction mixture for 1.5 hours, the reaction solution was adjusted pH=3-4 with 0.5 M dilute hydrochloric acid, the reaction solution was concentrated at 40-45°C under reduced pressure, methanol was evaporated, the concentrated mixture was filtered, the filter cake was eluted with a mixed solution of ethanol 3 ml water 6 ml, filtered, and the filter cake was dried to obtain compound VII.

### Example 3

The edoxaban tosylate intermediate 4,5,6,7-tetrahydro-5-methyl-thiazolo[5,4-c] pyridine-2-carboxylic acid of the present invention was obtained using the following process steps.

Compound (II), Compound (III), and Compound (IV-1) are shown in Example 1.

### (4) Synthesis of compound (V-3)

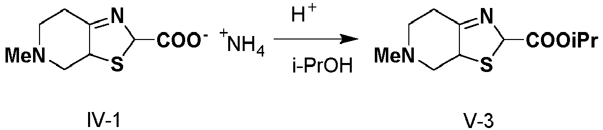

Into the reaction bottle were added isopropanol 10 ml, compound IV-1 crude 0.6 g, stirring to dissolve, controlling the temperature at 0-5 ° C, dropwise addition of concentrated sulfuric acid 0.5 ml (5 mmol), after finishing dropping, slowly rising the temperature to 20-25 ° C, holding the temperature and stirring the reaction mixture for 12 hours, using saturated sodium bicarbonate solution to adjust and neutralize pH = 7-8, the reaction mixture was concentrated at 50 ° C under reduced pressure , adding methanol 10 ml into the concentrated mixture and stirring, filtration, the filtrate was concentrated at 40 °C under reduced pressure to obtain the compound V-3 crude 0.55g.

### (5) Synthesis of compound (VI-3)

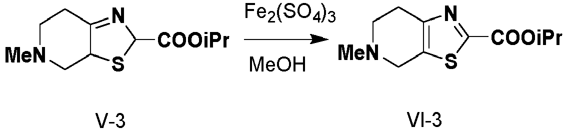

Into the reaction flask were added methanol 20ml, compound IV-3 crude 0.6g, Fe₂(SO₄)₃ solid 0.6g, stirring to dissolve, heating temperature, refluxing the reaction for 12 hours, filtration, the filtrate was concentrated at 50 °C under reduced pressure, the concentrated mixture was loaded on a silica gel column, solvent methanol / dichloromethane = 1:20 ratio of elution and purification, to obtain 0.22g compound VI-3.

### (6) Synthesis of compound (VII)

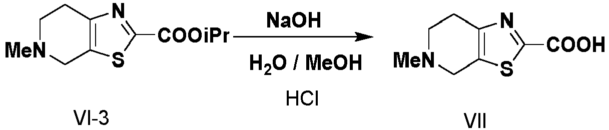

Into the reaction bottle were added 1.32 g (5.5 mmol) compound VI-3, methanol 10 ml, stirring to dissolve, the temperature was lowered to 0-5°C, dropwise addition of 1 M sodium hydroxide solution 6 ml (6 mmol), after finishing dropwise addition, maintaining the temperature at 0-5°C by heat preservation and stirring the reaction mixture for 1.5 hours, the reaction solution was adjusted with 0.5 M of dilute hydrochloric acid to pH = 3-4, the reaction solution was concentrated at 40-45°C under reduced pressure, the methanol was evaporated, the concentrate mixture was filtered, the filter cake was washed with a mixed solution of 3 ml of ethanol 6 ml of water, filtered, and the filter cake was dried to obtain compound VII.

### Example 4

The edoxaban tosylate intermediate 4,5,6,7-tetrahydro-5-methyl-thiazolo[5,4-c] pyridine-2-carboxylic acid of the present invention was obtained using the following process steps.

Compound (II), Compound (III), and Compound (IV-1) are shown in Example 1.

### (1) Synthesis of compound (VI-1)

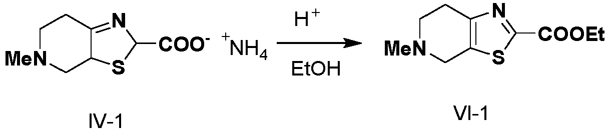

Into the reaction flask were added ethanol 20 ml, content of 47% of the crude compound IV-1 1.7 g (4 mmol), stirring to dissolve, controlling temperature at 20-25 ° C, dropwise addition of concentrated sulfuric acid 1.1 g (11 mmol), dropwise completion of the 20-25 ° C by insulation and stirring the reaction mixture for 24 hours, using saturated sodium bicarbonate solution to adjust and neutralize pH = 7-8, filtration, the filtrate was concentrated at 50 ° C under reduced pressure, the concentrated mixture was washed by adding ethyl acetate and water, stirring and layering, the organic phase was concentrated at 40 ° C under reduced pressure to obtain 0.7 g of compound VI-1, with a yield of 77.8%.

### (2) Synthesis of compound (VII)

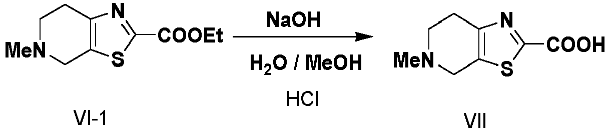

Into the reaction bottle were added 1.3 g (5.8 mmol) compound VI-1, methanol 10 ml, stirring to dissolve, the temperature was lowered to 0-5 ° C, dropwise addition of 1 M sodium hydroxide solution 6 ml (6 mmol), after finishing dropwise addition, maintaining the temperature at 0-5 ° C by heat preservation and stirring the reaction mixture for 1.5 hours, the reaction solution was adjusted with 0.5 M dilute hydrochloric acid to pH = 3-4, the reaction solution was concentrated at 40-45 ° C under reduced pressure, evaporated methanol, the concentrated mixture was filtered, the filter cake was washed with ethanol 3 ml/ water 6 ml mixed solution, after filtering, drying the filtrate cake to obtain compound VII.

### Example 5

The edoxaban tosylate intermediate 4,5,6,7-tetrahydro-5-methyl-thiazolo[5,4-c] pyridine-2-carboxylic acid of the present invention was obtained using the following process steps.

Compound (II), compound (III) were synthesized as shown in Example 1.

### (1) Synthesis of compound (IV-2)

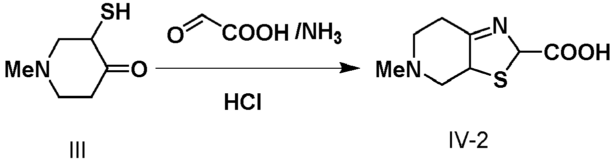

Into the reaction flask, 45g of methanol, 2.5g (27mmol) of glyoxalate monohydrate and 4g (27mmol) of Compound III were added, dissolved with stirring, the temperature was controlled to be below 30°C, and 7 M of ammonia/methanol solution of 16 ml (108mmol) was added dropwise, after dropping, the reaction mixture was stirred at 20-25°C for 24 hours while keep the temperature warm, the reaction was completed by adding hydrochloric acid dropwise to adjust the pH to 6.5, and the reaction liquid was concentrated at 40°C under reduced pressure to obtain 6.2g of solid crude Compound IV-2 in 48.5% yield.

### 2) Synthesis of compound (V-1)

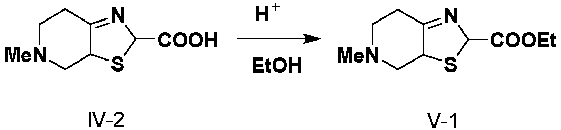

Into the reaction bottle were added ethanol 10 ml, compound IV-2 crude 0.6 g, stirring to dissolve, controlling temperature at 0-5 ° C, dropwise addition of concentrated sulfuric acid 1 ml (10 mmol), after dropping, slowly rising the temperature to 20-25 ° C by insulation and stirring the reaction mixture for 12 hours, the reaction mixture was saturated with sodium bicarbonate solution and neutralization of pH = 7-8, the reaction mixture was concentrated at 50 ° C under reduced pressure, the concentrated mixture was stirred by adding methanol (10 ml), after filtration, the filtrate was concentrated at 40 ° C under reduced pressure, to obtain the compound V-1 crude 0.6g.

(3) Compound (VI-1) as well as compound (VII) were synthesized as shown in Example 1.

### Example 6

The edoxaban tosylate intermediate 4,5,6,7-tetrahydro-5-methyl-thiazolo[5,4-c] pyridine-2-carboxylic acid of the present was obtained using the following process steps.

Compound (IV-2) was synthesized as shown in Example 5.

### (2) Synthesis of compound (VI-1)

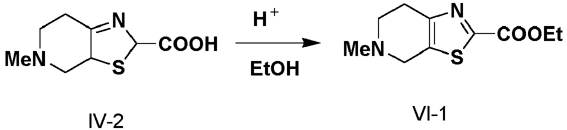

Into the reaction flask were added ethanol 20 ml, content of 50% of the crude compound IV-2 1.6 g (4 mmol), stirring to dissolve, controlling temperature at 20-25 ° C, dropwise addition of concentrated sulfuric acid 1.5 g (15 mmol), after finishing dropping, maintaining the temperature at 20-25 ° C, stirring the reaction mixture for 24 hours, using saturated sodium bicarbonate solution to adjust and neutralize pH = 7-8, filtration, filtrated at 50 ° C under reduced pressure concentration, the concentrated mixture was washed by adding ethyl acetate and water, stirring and layering, the organic phase was concentrated at 40 ° C under reduced pressure to obtain 0.73 g of compound VI-1, with a yield of 79.9%.

Compound (VII) was synthesized as shown in Example 1.

## Claims

1. An intermediate formula (IV) for the preparation of edoxaban tosylate, is **characterized by** the following structural formula: 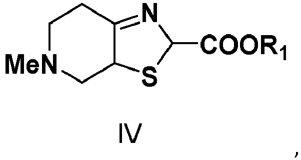 R₁=NH₄ or H.

2. A method of preparing an intermediate formula (IV) for edoxaban tosylate, **characterized in that** the synthesis step of the compound of formula (IV) comprises:
The compound N-methyl-4-piperidone was used as a raw material for the substitution reaction with bromine to produce the compound shown in formula (II),
The compound shown in formula (II) undergoes a thiolation reaction with sodium sulfide to obtain the compound shown in formula (III),
The compound shown in formula (III) was subjected to a cyclization reaction with glyoxalic acid to obtain the compound shown in formula (IV) with the following reaction formula: 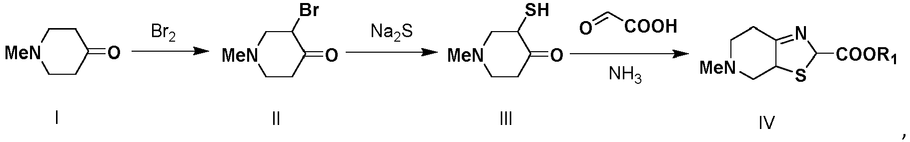
R₁=NH₄ or H.

3. An intermediate formula (IV-I) for the preparation of edoxaban tosylate, is **characterized by** the following structural formula:

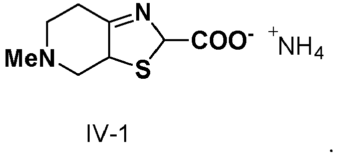

4. A method of preparing an intermediate formula (IV-I) for edoxaban tosylate, **characterized in that** the synthesis step of the compound shown in (IV-I) comprises: The compound shown in formula (III) was subjected to cyclization with glyoxalic acid as well as ammonia to give the compound shown in formula (IV-I) with the following reaction formula:

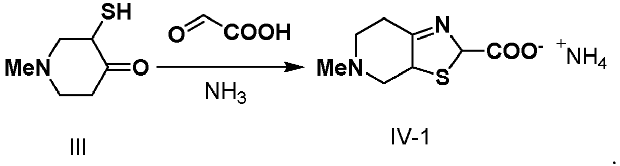

5. An intermediate formula (IV-2) for the preparation of edoxaban tosylate, is **characterized by** the following structural formula:

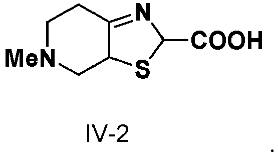

6. An intermediate formula (IV-2) for the preparation of edoxaban tosylate, wherein, the synthesis step of the compound shown in (IV-2) comprises: Acidification of the compound shown in formula (IV-I) to give the compound shown in formula (IV-2).

7. An intermediate formula (V) for the preparation of edoxaban tosylate, is **characterized by** the following structural formula: 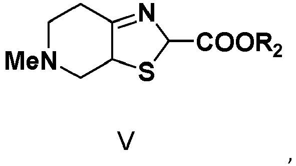 R₂ is selected from C1-C6 aliphatic hydrocarbons, phenyl, or substituted phenyl.

8. A method of preparing an intermediate formula (V) for the preparation of edoxaban tosylate, **characterized in that** the synthesis step of the compound shown in formula (V) comprises: The compound shown in formula (IV) undergoes an esterification reaction with an alcohol catalyzed by an acid to give the compound shown in formula (V) with the following reaction formula: 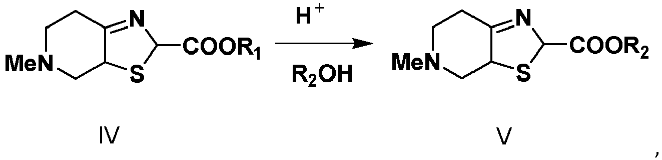 R₁ is selected from NH₄ or H, R₂ is selected from C1-C6 aliphatic hydrocarbons, phenyl, or substituted phenyl.

9. An intermediate formula (V-I) for the preparation of edoxaban tosylate, is **characterized by** the following structural formula:

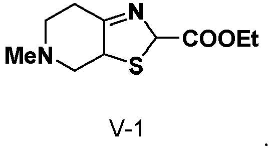

10. A method of preparing an intermediate formula (V-I) for edoxaban tosylate, **characterized in that** the synthesis step of the compound shown in (V-I) comprises: The compound shown in formula (IV-I) undergoes esterification reaction with ethanol catalyzed by acid to obtain the compound shown in formula (V-I) with the following reaction formula:

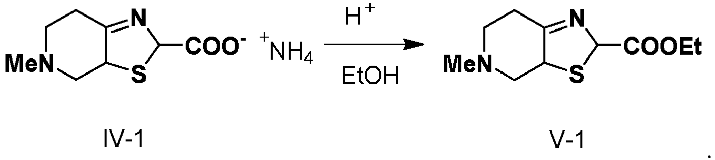

11. An intermediate formula (VI) for the preparation of edoxaban tosylate, is **characterized by** the following structural formula: 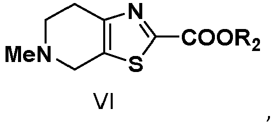 R₂ is selected from C1-C6 aliphatic hydrocarbons, phenyl, or substituted phenyl.

12. A method of preparing an intermediate formula (VI) for the preparation of edoxaban tosylate, **characterized in that** the synthesis step of the compound shown in formula (VI) comprises: The compound shown in formula (V) was obtained by esterification reaction of the compound shown in formula (IV) with an alcohol, and then the compound shown in formula (VI) was obtained by reaction, Or, the compound shown in formula (VI) is obtained directly from the reaction of the compound shown in formula (IV) in the presence of an alcohol and an acid with the following reaction formula: 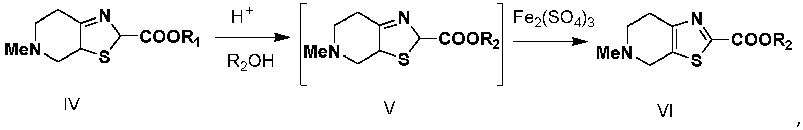 Or, 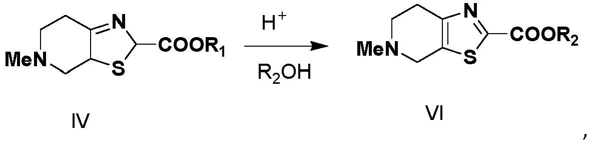 R₁ is selected from NH₄ or H, R₂ is selected from C1-C6 aliphatic hydrocarbons, phenyl, or substituted phenyl.

13. A method for the preparation of an intermediate formula (VI-I) for the preparation of edoxaban tosylate, **characterized in that** the synthesis step of the compound shown in formula (VI-I) comprises: The compound shown in formula (V-I) was obtained by esterification of the compound shown in formula (IV-I) with ethanol, and then reacted to obtain the compound shown in formula (VI-I), Or the compound shown in formula (VI-I) is obtained directly from the reaction of the compound shown in formula (IV-I) in the presence of ethanol and acid with the following reaction formula: 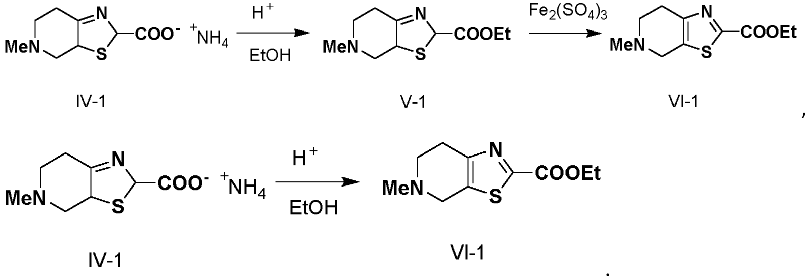 Or,

14. A method of preparing an intermediate formula (VII) for the preparation of edoxaban tosylate, **characterized in that** the synthesis step of the compound shown in formula (VII) comprises:
The compound N-methyl-4-piperidone was used as a raw material for the substitution reaction with bromine to produce the compound shown in formula (II),
The compound shown in formula (II) undergoes a thiolation reaction with sodium sulfide to obtain the compound shown in formula (III),
The compound shown in formula (III) was subjected to a cyclization reaction with glyoxalic acid to obtain the compound shown in formula (IV),
The compound shown in formula (IV) was esterified with an alcohol to obtain the compound shown in formula (V), which was then reacted to obtain the compound shown in formula (VI), Or, the compound shown in formula (VI) is obtained directly from the reaction of the compound shown in formula (IV) in the presence of an alcohol and an acid, The compound shown in formula (VI) was hydrolyzed by alkali and neutralized by acid to obtain the compound shown in formula (VII),
The reaction formula is as follows: 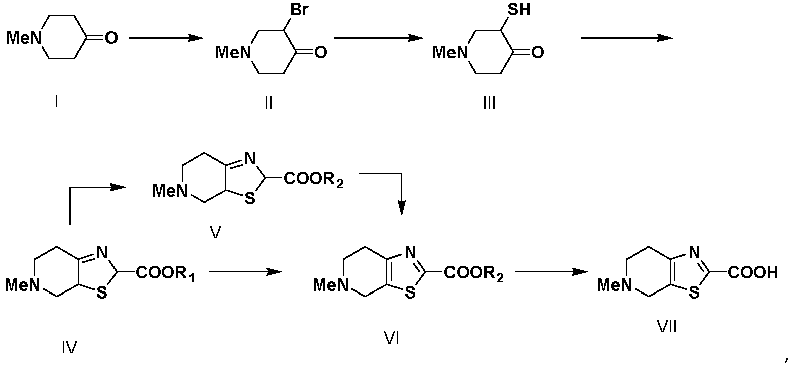 R₁ is selected from NH₄ or H, R₂ is selected from C1-C6 aliphatic hydrocarbons, phenyl, or substituted phenyl.

15. A method of preparing an intermediate formula (VII) for the preparation of edoxaban tosylate, **characterized in that** the synthesis step of the compound shown in formula (VII) comprises:
The compound N-methyl-4-piperidone was used as a raw material for the substitution reaction with bromine to produce the compound shown in formula (II),
The compound shown in formula (II) undergoes a thiolation reaction with sodium sulfide to obtain the compound shown in formula (III),
The compound shown in formula (III) was subjected to a cyclization reaction with glyoxalic acid to obtain the compound shown in formula (IV-I),
The compound shown in formula (IV-1) was esterified with an alcohol to obtain the compound shown in formula (V-I), which was then reacted to obtain the compound shown in formula (VI-I), Or,
the compound shown in formula (VI-I) is obtained directly from the reaction of the compound shown in formula (IV-I) in the presence of an alcohol and an acid,
The compounds shown in formula (VI-I) were hydrolyzed by alkali and neutralized by acid to give the compounds shown in formula (VII),
The reaction formula is as follows:
